# EUROPÄISCHE PATENTSCHRIFT

(11) **EP 0 790 845 B1**
(45) Veröffentlichungstag und Bekanntmachung des Hinweises auf die Patenterteilung: **19.05.1999**
(21) Anmeldenummer: 95936939.8
(22) Anmeldetag: 13.11.1995
(51) Int. Cl.: A61N 5/06

(54) **BEHANDLUNGSANORDNUNG FÜR LICHTTHERAPIE**
LIGHT THERAPY TREATMENT ARRANGEMENT
SYSTEME POUR PHOTOTHERAPIE

(30) Priorität: 11.11.1994 DE 4440112; 08.07.1995 DE 19524461
(43) Veröffentlichungstag der Anmeldung: 27.08.1997
(73) Patentinhaber: Optomed Opto Medical Systems GmbH, 12489 Berlin (DE)
(72) Erfinder: WILKENS, Jan, D-66424 Homburg (DE)
(74) Vertreter: Effert, Bressel und Kollegen
(86) Internationale Anmeldenummer: DE9501598
(87) Internationale Veröffentlichungsnummer: WO9614899

(56) Entgegenhaltungen:
- EP-A- 0 320 080
- EP-A- 0 324 490
- EP-A- 0 460 212
- EP-A- 0 478 506
- DE-A- 2 910 468
- DE-A- 3 121 689

## Beschreibung

Die Erfindung betrifft eine universelle Behandlungsanordnung zur Behandlung diverser Krankheiten, insbesondere auch von hyperproliferativen, malignen und entzündlichen Hauterkrankungen, der Psoriasis sowie weiterer Krankheiten und/oder zur kosmetischen Hautbehandlung sowie deren Verwendung, auch für Diagnoseverfahren.

Mit der DE-AS 17 64 685 ist bereits eine elektrische Allzweck-Entladungslampe mit einem lichtdurchlässigen Lampenkolben, einem an eine Spannungsquelle anschließbaren Elektrodenpaar, einer ionisierbaren Füllung und einem auf der Kolbeninnenwand aufgebrachten Leuchtstoffbelag vorgeschlagen worden, mit der im Bereich oberhalb 290 nm Wellenlänge, insbesondere für Mittelultraviolettstrahlung eine Leistung von 6 bis 50 µW pro Lumen sichtbaren Lichts und für den Bereich des Nahultraviolett eine Leistung von 150 bis 700 µW pro Lumen sichtbaren Lichtes erzeugt werden soll, wobei die Leistung in den beiden Bereichen im Verhältnis von 1:8 bis etwa 1:40 gestaltet sein soll. Zusätzlich ist definiert, daß die Gesamtstrahlung pro Lumen etwa denselben Anteil haben soll, wie natürliches Tageslicht entsprechender Farbtemperatur. Abgesehen davon, daß der Leistungsvergleich lm/Watt irreführend ist und hier unsichtbares zu sichtbarem Licht ins Verhältnis gesetzt wird, errechnet sich, daß für den Gesamtbereich von 290 bis 320 nm eine maximale Leistung von 1,5 mW und für den Bereich von 320 bis 380 nm eine maximale Leistung von 42 mW emittiert werden soll. Ein Flächenbezug ist nicht angegeben.

Weiterhin ist offenbart, daß derartige Lampen bis in den Bereich von 700 nm unterschiedliche Maxima z.B. bei 570 bis 595 oder 595 bis 625 nm haben können. Insgesamt wird in der Schrift auch Stand der Technik diskutiert, der sich mit Leuchtstofflampen spezieller Wellenlänge beschäftigt, die im Gegensatz zum Tageslicht ein scharfes Maximum bei verschiedenen Wellenlängen haben können. Dafür sind entsprechende Leuchtstoffmischungen angegeben, wobei charakteristischen Metallverbindungen für verschiedene Farbtemperaturen aufgelistet sind. Neben den erwähnten speziellen Verhältnissen im Bereich des UV-Lichtes sind für Lampen mit 40 W Nennleistung und einer Lichtleistung von 2100 bis 2300 lm die verschiedenen Leistungen natürlichen Lichtes gegenüber ausgewählten künstlichen Lampen dargestellt. Ziel der Erfindung ist es dort, eine Lampe vorzuschlagen, die während des 8 stündigen Arbeitstages eine unerwünschte Rötung der Haut (analog Sonnenbrand) vermeidet und trotzdem einen erwünschten Farbwiedergabeeffekt hat. Erwähnt sind auch verschiedene biologische Wirkungen in tabellarischer Form, insbesondere die Einwirkung von Licht auf den Augenfarbstoff, die Wirkung auf Zirbeldrüsen und Keimdrüsen durch Bestrahlung innerhalb einer Bereiches von 380 bis 700 nm Wellenlänge sowie die bakterientötende Wirkung von UV-Licht mit 254 nm Wellenlänge . Für den UV-Bereich ist insbesondere die Bildung von Vitaminen, die Inaktivierung von Mikroorganismen sowie die kosmetische Wirkung erwähnt. Hingewiesen wird ebenfalls darauf, daß UV-Strahlung Änderung in den Melaninmengen der Haut hervorrufen kann; dieser Effekt wird jedoch insbesondere der Einwirkung von Licht der Wellenlänge 290 bis 320 nm zugeschrieben und daher hervorgehoben, daß für diesem Bereich eine geringere Strahlung von den erfindungsgemäßen Allzwecklampen mit natürlichem Tageslichtspektrum vorzusehen sei.

In gleicher Weise wird in der DE 23 39 181 C 2 vorgeschlagen im sichtbaren Spektralbereich von 300 bis 800 nm mit angegebenen Einzel- und Zwischenbereichen eine relativ homogene Engergieverteilung über die einzelnen Spektralbereiche vorzuschlagen. Eine derartige Lampe soll für die Pflanzenzucht verwendet werden, indem natürliches Sonnenlicht nachgeahmt wird.

Mit der DE 31 21 689 C 2 ist eine Leuchtstofflampe vorgeschlagen worden, die im Bereich von UVC und UVB durch den Glaskolben filternd wirkt. Für den Bereich von UVA ist angegeben, daß hier insbesondere ein Maximum oberhalb 350 nm mit einer Spektral-Breite von etwa 320 bis 400 nm gegeben sei und zusätzlich durch einen zweiten Leuchtstoff eine ausgeprägte Strahlungsemission im Orange-Rot-Bereich bei etwa 650 nm vorgesehen ist. Dies soll bewirken, daß üblicherweise zur Bräunung oder zur Behandlung von Psoriasis eingesetzte Leuchstofflampen in Solarien (DE-OS 26 28 091) keine Nebenerscheinungen wie Müdigkeit oder verminderte Aktivität erzeugen. Durch den Orange-Bereich soll eine einseitige Beeinflussung des Nerventonus und eine Gefäßweitstellung hervorgerufen werden, um Müdigkeit vorzubeugen. Allerdings ist angegeben, daß eine derartige Lampe im Bereich 404 und 437 nm (blau) ein für Quecksilber typisches Emissionsmaximum haben und daher es nicht möglich sei, den Blau-Bereich auszufiltern, was nach der Darstellung in der Beschreibung erwünscht ist.

In der DE-OS 34 31 692 wird vorgeschlagen, eine sonnenlichtähnliche Leuchtstofflampe mit fünf Energiemaxima bei etwa 320, 380, 450, 550, 650 nm zu verwenden, welche durch entsprechend dotierte Lampen mit den angegebenen Leuchtstoffen in einer Mischung erzielt werden sollen. Erwähnt ist, daß im UVA-Bereich Zellschäden reparabel sind und eine Regeneration der Augen ermöglicht wird, während im Bereich unterhalb 320 nm die langwellige UVB-Strahlung die Bildung des Vitamin D 3 und eine Calziumresorption, Aktivierung des Stoffwechsels und Leistungssteigerung der Muskulatur und Kreislauforgane bewirke. Eine Sonnenbrandwirkung sei nur mit Wellenlängenbereichen unter 300 nm verbunden. Für den Bereich von 300 bis 400 nm werden etwa ähnliche Spektralemissionen angegeben, wie in der zuvor zitierten DE-AS 17 64 685.

In einer Vielzahl von Schriften sind für die unterschiedlichen Strahlungsbereiche, z.B. für rot bis dunkelrot (US-PS 32 87 586) oder für den blauen Bereich (DE-OS 19 22 416) geeignete Dotierungsmetalle angegeben. Spezielle Lampen (DD 201 107 und DD 221 374) wurden für therapeutische Fenster um 325 nm Wellenlänge konzipiert. Für die Behandlung von Hautstörungen ist in der DE-OS 32 39 417 eine Phosphor-Dotierung für eine optimierte Emission bei 340 - 400 nm vorgeschlagen worden.

In der DE 29 10 468 A 1 wird eine UV-Leuchtstoff-Strahlungsquelle für fotobiologische und fotochemische Zwecke, insbesondere zur Bräunungsbestrahlung vorgeschlagen, bei der der Kolben der Strahlungsquelle in Berührung mit einer Flüssigkeitschicht steht, vorzugsweise einer Wasserschicht oder einem Wasserbad. Ein solches Wasserbad kann auch als Wasserbett mit einer Liege ausgestaltet sein, wobei die Projektionsfläche, eine flexible transparente Abdeckung des Wasserbettes ist und die Wassertemperatur auf 30 bis 50°, vorzugsweise 35 bis 40° eingestellt werden soll. Dazu wird im Detail eine Anordnung der einzelnen Strahlungsquellen vorgeschlagen, die in rinnenförmigen Wasserbehältern, welche die Leuchtstoffröhren umschließen, angeordnet sind. Diese rinnenförmigen Elemente sollen mit Reflektoren auf der dem Bestrahlungsobjekt abgewandten Seite versehen sein, um eine Lichtreflektion von dieser Kühleinrichtung in Richtung Projektionsfläche zu ermöglichen. Vorgeschlagen wird dabei die Verwendung von UV-Leuchtstofflampen im Spektrum von 300 bis 430 nm mit thermisch nicht hochbelastbaren Leuchtstoffen, gegebenenfalls sollen dem Kühlwasser Zusätze zur Beeinflussung des spektralen Transmissionsgrades und/oder der Herabsetzung der elektrischen Leitfähigkeit beigesetzt werden. Als erzielbare pigmentierungswirksame Strahlungsleistung, d.h. Bestrahlungsleistung im genannten UV-Bereich soll bei etwa 140 W/m² liegen. Fakultativ ist erwähnt, daß diese Bestrahlungseinrichtung auch für die medizinische Applikation (Diagnose und Therapie) geeignet ist, insbesondere auch für die Behandlung von Hautkrankheiten und Hautschäden. Das Gerät hat sich nicht durchsetzen können, weil der Hauptzweck, die Bräunung, mit Solarien herkömmlicher Art billiger erreichbar ist und das Gerät in der medizinischen Praxis zu umständlich und zu teuer war. Ein Hauptgrund mag darin liegen, daß die Lampen sehr stark beansprucht wurden, da eine Oberflächentemperatur der Lampe von 35 bis 40° C insbesondere bei einer relativ hohen Nennleistung von 115 W die Lebensdauer der Lampen herabsetzt.

Für Infrarothochleistungsstrahler bei der Hyperthermietherapie ist bereits vorgeschlagen worden (DE 40 33 958 A1), eine Kühl- und Filtereinrichtung vorzusehen, die aus einem zum Strahler angeordneten koaxialen Mantelrohr besteht, durch das Wasser an der Strahleroberfläche entlang geführt wird. Das Wasser kann Farbstoffgehalte zur Absorption bestimmter Strahlungen, hier insbesondere der sichtbaren Strahlung zu Vermeidung von Blendungen des Patienten enthalten; zugleich können dieser Kühlflüssigkeit auch antibakterielle Substanzen beigegeben werden.

Die Psoriasis oder Schuppenflechte ist eine Ganzkörpererkrankung. Zu ihrer Behandlung wird derzeit als modernste und wirkungsvollste Therapie die Photochemotherapie eingesetzt. Sie beruht auf dem Umstand, daß sich Psoralen (oral vor der Behandlung verabreicht) unter Einwirkung von ultravioletten Licht (UV-A, 365 nm) mit der DNS der Epidermiszellen zu Photoaddukten mit Quervernetzung des Doppelstranges verbinden und so eine Zellteilung unmöglich macht. Ein weiterer bekannter Gedanke besteht darin, eine Bestrahlung allgemein mit UV-B-Licht vorzunehmen, bei dem schnellteilende Zellen zugrunde gehen. Die Zellen der Schuppenflechte teilen sich etwa 5-fach schneller als gesunde, normale Zellen, so daß eine Bestrahlung sehr wirkungsvoll ist. Der Nachteil beider Gedanken besteht in dem erhöhten karzinogenen Risiko, also der erhöhten Krebsgefahr, insbesondere bei Langzeitanwendung aufgrund der Bestrahlung mit ultraviolettem Licht.

Um diese Nachteile zu umgehen, ist bereits vorgeschlagen worden, photosensitive Zellen durch Photosensibilisatoren in einem anderen Bereich des Spektrums zu schaffen, also im nichtkarzinogenen Bereich. In der US-PS 4 753 958 wird der Einsatz von Hämatoporphyrinen vorgeschlagen. Diese Substanzen reichern sich in hyperproliferativem Gewebe an, also z.B. in Tumor- oder Entzündungsgewebe. Diese "Prodrugs" wandeln sich in Porphyrin in der Zelle um. Nach ausreichender Belichtung des so angefärbten Gewebebereiches kommt es zu einer Photolyse dieses Farbstoffes. Diese Photolyse führt nun zu einer intrazellulären Freisetzung von zelltoxischen Bestandteilen. Durch die Bestrahlung werden also ganz gezielt die befallenen, erkrankten Zellgewebe angesprochen und so durch einfache Belichtung zerstört.

Die Bestrahlung erfolgt regelmäßig mit Hochleistungsglühlampen, bei denen mittels Rotfiltern die unerwünschten Spektralbereiche herausgefiltert werden. Die Lampen zeichnen sich trotz einer hohen Verbrauchsleistung nur durch geringe Wirkleistung bei einer gleichzeitig sehr hohen Wärmeentwicklung aus. Dies macht die Behandlung relativ kostenintensiv und darüber hinaus auch für den Patienten unangenehm und beschränkt darüber hinaus die Dauer der möglichen Belichtung. Auch ist eine Ganzkörperbehandlung praktisch ausgeschlossen, wenn nicht aufgrund der hohen Wärmeentwicklung eine "grillartige" Wirkung auf den Patienten ausgeübt werden soll. Die mangelnde Strahldichte der UV-Lampen soll gemäß der DD 251 707 durch Nutzung der Abwärme kompensiert werden, indem diese als Warmluftstrom mittels Gebläse auf die Haut gerichtet wird. Patienten werden dies wegen der Geräuschbelästigung und Strömung selten als angenehm empfinden.

Alternativ sind auch bereits leistungsstarke Farbstofflaser eingesetzt worden, die aber enorm hohe Anschaffungskosten verursachen und darüber hinaus noch kleinere Areale ausleuchten. Auch die Anwendung von 5-δ-Aminolävulinsäure im Zusammenhang mit einer solchen Lasertherapie bei 635 nm Wellenlänge ist gelegentlich schon vorgeschlagen worden.

Von daher liegt der Erfindung das Problem zugrunde, eine deutlich kostengünstigere und einfachere, aber bei vielen Krankheiten universell anwendbare Bestrahlungsanordnung und deren Verwendung zur spezifischen Licht-Behandlung vorzuschlagen.

Das Problem wird durch die Merkmale der Ansprüche 1, 14 und 15 gelöst. Weiterbildungen der Erfindung sind in Unteransprüchen erfaßt.

Durch diese Lösung wird eine realisierbare und kostengünstige Bestrahlungsanordnung für Psoriasis-Patienten und eine Vielzahl weiterer genannter Therapien möglich. Die Strahlungsanordnung ist darüber hinaus sowohl für Ganzkörpererkrankungen als auch für vertikales Standgerät oder als hängendes sowie für auf bestimmte Körperteile gerichtetes Gerät gestaltet sein. Bei Geräten die Stehen oder Sitzen des Patienten erlauben, kann die Patientenruheeinrichtung auch drehbar vor der Bestrahlungseinrichtung ausgeführt werden.

Behandelbar mit der Erfindung sind insbesondere hyperproliferative Epithelerkrankungen, die sich durch epidermale Zellproliferation und unvollständige Zelldifferenzierung auszeichnen. Hierzu gehören Akne, Psoriasis, prämaligne und maligne Epithelveränderungen, z. B. Dysplasie, Herpes simplex, Warzen, Ichtyosis, sonneninduzierte Keratose, nichtmaligne Keratose, Plattenepithelkarzinom, Basalzellkarzinom, Melanom. Ferner können behandelt werden Neurodermitis, atopische Dermatitis, Kontaktdermatitis, Vitiligo.

Die Erfindung macht sich dabei die Vorteile die bisherigen sehr zahlreichen Lichttherapie-Versionen zunutze und verbessert die Therapie-Varianten unter Vermeidung der bekannt gewordenen Schwächen.

Leuchtstofflampen sind relativ kostengünstig. Darüber hinaus ist es im Gegensatz zu den Glühlampen nicht erforderlich, mittels Filtern bestimmte Bereiche des Spektrums auszublenden (und in Wärme zu verwandeln), sondern es wird durch geeignete Dotierung der Spektralbereich der abzugebenden Strahlung in den gewünschten Bereich verschoben, hier z.B. in den sichtbaren Bereich zwischen 400 nm und 800 nm oder den bevorzugten UVA 1 bis Blau-Bereich.

Besonders geeignet haben sich hierbei Leuchtstofflampen mit Amalgan- bzw Amalgan-Indium- Füllung sowie Dotierungen mit Magnesiumfluorgermanat oder Yttriumvanadat oder Cer-Terbium-Magnesiumaluminat oder Lithiummetallaluminat gezeigt. Diese geben eine Strahlung im jeweils gewünschten prinzipiellen Spektralbereich ab, der durch alternative Zusammensetzungen schmalbandig gestaltet werden kann. Eine entsprechende Dotierung der Leuchtstoffröhren mit diesen Materialien ist auch im kostenmäßig tragbaren Bereich, insbesondere im Hinblick auf die bei herkömmlichen Anlagen anfallenden Kosten. Verwendet werden dabei auch die für bestimmte Spektralbereiche für sich schon bekannten Dotierungsmaterialien.

Im gezielten Spektralauswahlbereich ist es darüber hinaus bevorzugt, wenn die Leuchtstofflampen eine spezifische Licht-Leistung von mehr als 700 W/m² emittieren, von denen etwa 500 W/m² das Bestrahlungsobjekt erreichen. Diese relativ hohe Leistung kann die Behandlungsdauer einer einzelnen Behandlung deutlich verkürzen und ist im Gegensatz zur herkömmlichen Behandlung mit breitbandigem ultraviolettem Licht auch für den behandelten Patienten tragbar, da praktisch ausschließlich die befallenen, erkrankten Hautpartien auf die Strahlung reagieren, nämlich durch die Photolyse zerstört werden. Natürlich ist eine Abschirmung der Augen dabei sinnvoll.

Eine Leistungssteigerung für eine am Bestrahlungsobjekt wirksame spezifische Leistung wird dabei durch eine in oder auf der Lampe an der Wandung aufgebrachte Reflexionseinrichtung z. B. einer Folie oder Reflexionsbedampfungsschicht erreicht.

Prinzipiell werden Lampen mit einem sehr geringem Durchmesser von z.B. 12 mm oder 6 mm verwendet die in geringem Abstand in die Module eingesetzt werden und die von einem Kühlmedium in gerichteter Strömung umspült werden.
Die Lampen werden mit für ihren Durchmesser übergroßen Wendeln bestückt und ereichen dadurch eine höhere Strombelastbarkeit.
Es werden elektronische Vorschaltgeräte verwendet, die bei 25 kHz statt der früher üblichen 50 Hz arbeiten. Dadurch wird die Blindleistung auf ein Minimum reduziert.
Mit einem dem Dimmer ähnlichen Strom- oder Spannungssteller kann die Leistung zusätzlich stufenlos der Verwendungsart z.B. beim Wechsel von erwachsenen Patienten zu Kindern angepaßt werden.
Alle Maßnahmen zusammen in Kombination mit einer gezielten Oberflächenkühlung ermöglichen eine Vervielfachung der Emissionsleistung in den für die Bestrahlung erfindungsgemäß wichtigen Bereichen auf etwa 700 W/cm² und höher. Die Strahlleistung wird dabei bei der normierten Wellenlänge von 555 nm errechnet. Versuche in der Ulbricht-Kugel und mit weiteren Meßapparaturen haben die erwarteten Ergebnisse bestätigt.

Die Kühlung der Leuchtstofflampen hat mehrere Vorteile: Einerseits führt sie die Verlustleistung aus dem Fluoreszenzfarbstoff und dem Glas der Leuchtstofflampe ab, so daß eine erhöhte Abstrahlleistung bei moderaten Temperaturen in allen Teilen der Leuchtstofflampe erreicht wird. Andererseits verhindert die Kühlung die Abstrahlung von langwelliger Wärmestrahlung von der Lampe zum Patienten.
Durch Thermostatisierungen der Kühlflüssigkeit kann eine Optimierung der Abstrahlungsdaten und/oder einer Verbesserung des Wärmetauschs Patient/Umgebung mit dem Zweck der Steigerung des Wohlgefühls des Patienten erreicht werden. Dabei wird zum einen die Umgebungstemperatur der Lampe auf den für maximale Leistung optimalen Temperaturbereich von 8 bis 20 Grad Celsius mit Spreizbreite von max. 5 bis 30° C eingestellt und zum anderen zumindest der Kontaktbereich des Patienten in der Ruheeinrichtung auf eine höhere Temperatur aufgewärmt. Die Kühlung der Leuchtstofflampe kann dabei direkt oder indirekt erfolgen.

Direkte Kühlung bedeutet, daß die Leuchtstofflampe, außer im Bereich ihrer elektrischen Anschlüsse, vollständig in das Kühlmittel eingetaucht betrieben werden. Dabei kann die Bestrahlungseinrichtung wannenartig ausgebildet sein, so daß die Nutzstrahlung durch den Flüssigkeitsspiegel des Kühlmittels nach oben abgestrahlt wird. Der Patient liegt in diesem Fall auf einem transparenten oder perforiertem Tuch oberhalb der Kühlmittelwanne. Das Tragetuch kann auch als textiles Netz ausgebildet sein.

Alternativ kann die Bestrahlungseinrichtung auch so ausgeführt werden, daß ihre Abstrahlfläche mit einer flüssigkeitsdicht aufgebrachten transparenten Abdeckung, z.B. Glas abgeschlossen ist. Das Innere der Bestrahlungseinrichtung ist dann vollständig mit dem Kühlmittel gefüllt, dadurch ist eine Bestrahlung in beliebiger Lampenrichtung möglich.

Auch indirekte Kühlung ist möglich, wobei die Leuchtstofflampen nicht in die Kühlflüssigkeit eintauchen, sondern hier die Wärme durch Strahlung, Wärmeleitung und Konvektion zu einem Lampenkühler abgeführt wird, der seinerseits von Kühlmittel durchströmt wird oder eine Kühlfläche zur Wärmeabgabe an die Umgebung aufweist. Zusätzlich kann die Leuchtstofflampe im Bereich der Elektroden in einen Kühlkörper aus gut wärmeleitenden Material eingebaut werden, der die Leuchtstofflampe auf einem großen Teil ihres Umfanges innig berührt und die absorbierte Wärme zum Lampenkühler hin ableitet.

Zur Vermeidung der Bestrahlung des Patienten mit langwelliger Wärmestrahlung kann ein besonderer Strahlungskühler zwischen Leuchtstofflampe und dem Patienten angebracht werden. Dieser besteht aus zwei parallel angeordneten Scheiben aus Glas oder einem anderen für die Nutzstrahlung transparenten Material. Der Zwischenraum zwischen den genannten Platten ist von Kühlmittel z.B. speziellen Gasen oder wäßrige Fluide durchströmt und dazu mit geeigneter Abdichtung, Halterung sowie Zu- und Abflußöffnungen versehen.

Für die Behandlung von Psoriasis und Hauttumoren bietet bei den erfindungsgemäß erreichbaren Bestrahlungsdichten der rote (600-700 nm), grüne (500 bis 570 nm) unter UV-A-1/blaue (360-460 nm) Spektralbereich, vorzugsweise in Verbindung mit einer Synthesesteigerung körpereigene Porphyrine durch oberflächlichen Behandlung von Amino-delta-Lävulinsäure, incl. deren Ester und/oder Glucose, incl. deren Ester und/oder Eisenverbindungen in Form oraler Applikation oder entsprechender Salben Vorteile.

Für die Behandlung von Pruritus z.B. bei der Hyperbilirubinämie und Niereninsuffizienz bietet bei den erreichbaren Bestrahlungsdichten der blaue (400-500 nm) und der grüne (500 bis 600 nm) Spektralbereich Vorteile.

Insbesondere beim Neugeborenen-Ikterus ist im grünen Bereich die nützliche Leistung erhöhbar, da nur bei blauen Ausläufern durch mögliche schädliche Wirkungen die Dosisleistungserhöhung begrenzt.

Für die Behandlung von Akne, Asthma bronchiale, Lupus erythematosus, Neurodermitis und Psoriasis bietet bei den erfindungsgemäßen Bestrahlungsdichte der Spektralbereich blau bis UV Vorteile, insbesondere UV-A-1. Bei Akne sind endogene Porphyrine (von Bakterien synthetisiert) nachweisbar, und die Photolyse dieser Porphyrine beeinflußt das bakterielle Wachstum, sodaß nicht, wie bisher üblich, der gesamte UV-Spektralbereich für die Lichttherapie zur Verfügung steht.

Mit sehr hohen Bestrahlungsintensitäten vorzugsweise im grünen Bereich zwischen 500 und 600 nm (bis zu 700 J/cm²), ist es möglich eine erhebliche Beeinflussung von Immunfunktionen zu erreichen, die eine günstige Wirkung auf die genannten Erkrankungen haben.

Die Behandlung von Hypertonie und Durchblutungsstörungen bietet bei den erreichbaren Bestrahlungsdichten der Bereich zwischen 600 und 800 nm Vorteile.

Für die Behandlung von Depressionen, vorzugsweise bei saisonalen abhängigen Erkrankungen (Winterdepression) und Schizophrenien, Folge der Schichtarbeit, Delayed Sleep Phase Syndrom, Jet-Lag Syndrom, Prämenstruelles Syndrom, Alkoholentzugssyndorm hat sich weißes Licht in hoher Intensität, aber auch blaues und grünes Licht als wirksam erwiesen. Die positiven Effekte werden durch gleichzeitige Einwirkung von einem Magnetfeld verstärkt.

Da die photobiologischen Effekte einer nicht-linearen Wirkungsfunktion unterliegen, kann erst die Bestrahlung mit einer ausreichend hohen Intensität die beschriebenen positiven Wirkungen beim Patienten auslösen und nicht eine entsprechend verlängerte Bestrahlung mit derzeit verfügbaren niedrigen Intensität.

Besonders bevorzugt ist es, wenn eine Hautphotosensibilisierung z.B. mit einer Salbe auf Basis von 5-δ-Aminolävulinsäure erfolgt. Diese Substanz hat sich gerade in diesem Zusammenhang als ganz besonders vorteilhaft erwiesen. Generell sind systemisch oder topisch anwendbare Photosensibilisatoren für die Erfindung bevorzugt, die im nicht mutagenen Spektralbereich oberhalb von 400 nm wirksam sind. Noch mehr bevorzugt ist es, wenn von der Substanz ein Äthyl-oder Metylesterderivat verwendet wird. Versuche haben gezeigt, daß diese Esterderivate der Substanz - ohne Penetrationsvermittler - eine noch erheblich verbesserte Gewebseindringungstiefe bewirken (eine größere Lipophilie) und sich deshalb noch besser als Sensibilisator an Oberflächen eignen als die Ursprungssubstanz. Denkbar ist dadurch sogar eine kurative Bestrahlung von Hautkarzinom, u.U. sogar prophylaktisch.

Die Verwendung von Leuchtstofflampen, die oberhalb des UV-Bereiches emittieren, zur Behandlung von hyperproliferativen, malignen und/oder entzündlicher Hauterkrankungen, z.B. der Psoriasis, ist bisher noch überhaupt nicht in Betracht gezogen worden. Die erhebliche Verbesserung der Bestrahlungsmöglichkeiten, insbesondere die deutliche Verminderung des karzinogenen Risikos bei gleichzeitiger Vermeidung von Unzuträglichkeiten aufgrund von Wärmeentwicklung läßt gerade bei einer häufigen, an sich unheilbaren Hauterkrankung die wesentlichen Vorteile dieser Erfindung bedeutend erscheinen, ganz abgesehen von den gesundheitsökonomischen Möglichkeiten der Erfindung.

Da die Substanzen wie Aminodeltalävulinsäure und auch ihre Esterderivate selbst nicht photosensitiv sind, lassen sie sich als Salbe sehr gut benutzen, da trotz der massiven Bestrahlung kein "Sonnenbrand" oder ähnliche Effekte auftreten können. Die gesamten Effekte treten nur bei der in die Zellen gelangten umgewandelten Substanz, nämlich dem körpereigenen Protoporphyrin IX ein.

Methylenblau (Pyoktonin) und verwandte Phenothiazinium Farbstoffe sind ebenfals als äußerlich anzuwendende Fotosensibilisatoren für die Haut geeignet.

Als Arzneimittel/Salbe die Eisenverbindungen enthalten kommen eine große Anzahl dreiwertiger komplexer Verbindungen infrage, beispielsweise Ferrum sulfuricum, Ferrum chloratum oder Eisen-Sorbit-Citrat-Komplex. Bevorzugt sind durch Zusatz von Ascorbinsäure gegen Oxidation schützbare Ferroverbindungen. Die Resorption kann durch Verwendung von Eisensalzen organischer Säuren wie Aspartat, Fumarat, Succinat und ähnliche gefördert werden. Vorteilhaft ist auch die oberflächliche Anwendung von Hämeisen aus dem Myoglobin des Fleisches oder des Blutes sein, da dann das Häm-Eisen von Epithelzellen als intaktes Molekül aufgenommen wird und in der Zelle das Eisen freisetzt.

Unter zuckerhaltigen Verbindungen werden auch Zuckeralkohole und deren Ester, Glucose, Fructose, Sorbit in einfacher und veresterter Form, Sacharide, Zuckersäuren, Zuckerphosphate und deren komplexes Lösungsgemisch verstanden.

Die äußerliche Anwendung der zuvor genannten fotosensibilisierenden Wirkstoffe als fertige Präparate wird durch entsprechende Grundlagen (Trägerstoffe), deren Kombinationen und Zusatzstoffe (Wirkstoffe), die in die Grundlagen eingearbeitet werden ermöglicht.
Als Grundlagen kommen infrage Eucerinum anhydricum, Vaseline, Lanolin und Schweineschmalz, außerdem synthetische Fette wie Carbowax und Polyethylenglykol. Mit einer aufgetragenen Fettschicht wird ein künstliches Spatium zwischen Fettschicht und Haut geschaffen, wodurch sich die Wirkstoffe besser verteilen und leichter in die Tiefe dringen. Der Zusatz von Emulgatoren verleiht den wasserabweisenden Grundstoffen die Fähigkeit zur Wasseraufnahme.
Der Auftrag kann je nach Hauttyp als Emulsion (Creme mit Wasser in der Innenphase oder Wasser in der Außenphase) erfolgen. Darüberhinaus können wasserlösliche Salbengrundlagen aus Polyehtylenglykol oder quellenden Kolloiden in Glycerinwasser verwendet werden.
Die derart präparierte Haut reagiert günstig auf die applizierte spektralspezifische Bestrahlung mit den Leuchtstofflampen der erfindungsgemäßen Art.

Durch die großflächige Ausleuchtung durch die Leuchtstofflampen im Gegensatz zu den vor allem bei Laserlicht auftretenden punktförmigen Lichtquellen ist eine sehr viel gleichmäßigere räumliche Lichtverteilung zu erwarten. Bei entsprechender Verwendung wäre daher auch eine Diagnose lediglich tumorverdächtiger Porphyrinanreicherungen möglich.

Eine weitere erfindungsgemäße Ausführungsform setzt mehrere Leuchtstofflampen mit unterschiedlichen Spektralbereichen, also beispielsweise rot und/oder grün und/oder blau für einen speziellen Zweck ein. Die einzelnen Farben werden dabei abwechselnd mit etwa 100 Hz geschaltet. In Verbindung mit einer Rot-Filter-Brille mit LCD-Shutter wäre dann die zeitgleiche Darstellung mit 50 Hz pro Auge für den behandelnden Arzt mit Anregungswellenlängen unterschiedlicher Gewebseindringungstiefe möglich. Hierdurch wäre die mögliche Tiefenausdehnung von Hautveränderungen sichtbar und diagnostisch zu nutzen.

Für kosmetische Behandlungen sind erfindungsgemäße Bestrahlungsanordnungen sehr praktisch; sie können auch von Sonnenbräunungsstudios für die gezielte kurzzeitige Bräunung verschiedener Körperteile durch Auswechseln von Modulen eingesetzt worden. Durch die Erfindung wird nun erstmals eine gezielte Ganzkörperbestrahlung ohne risikobehaftete Nebenwirkung möglich; durch den Einsatz der Leuchtstofflampen kommt es zu einer beschleunigten Klärung der unerwünschten Hautveränderung und damit zu einem nennenswerten positiven kosmetischen Effekt.

Anhand einer Zeichnung werden Ausführungsbeispiele der Erfindung näher erläutert. Es zeigen:
- Fig. 1, 1a, 1b: eine hängende Behandlungsanordnung mit Details in Seitenansicht und Teilschnitt;
- Fig. 2: eine zweite Ausführung einer hängenden erfindungsgemäßen Anordnung;
- Fig. 3: eine Standversion der erfindungsgemäßen Anordnung.

Im folgenden werden dieselben Bezugsziffern für identische oder gleichwirkende Teile verwendet.

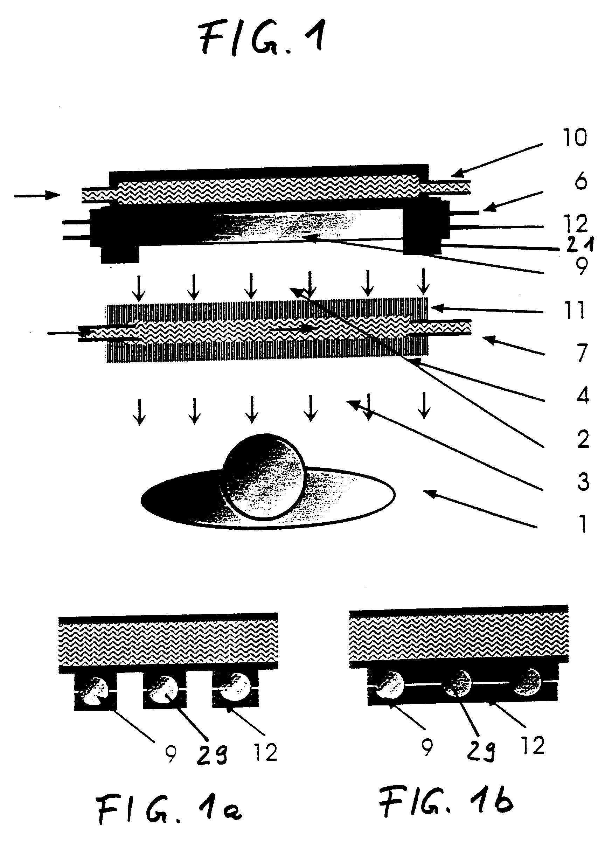

In einem unmaßstäblich dargestellten Modulrahmen 21 sind Leuchtstofflampen 9, 29 angeordnet, deren Dotierung so abgestimmt ist, daß die Lampe 9 ihr Leistungsmaximum bei 370 nm und die Lampe 29 bei 410 nm hat. Beide Lampenkolben haben 12mm Durchmesser und 60 W Anschlußleistung; ihre Strahlleistung beträgt an der Oberfläche 710 W/m².
Das nicht gezeigte frequenzregelbare elektronische Vorschaltgerät ist mit einem Dimmer versehen, mit dem die Strahlleistung und/oder die Stromaufnahme stufenlos regelbar sind. In einer alternativen Bauform (Fig. 1b) ist auch die Spannung in gewissen Grenzen regelbar.
Diese Anordnung hat in der Praxis die Möglichkeit geschaffen, die Spannung bei Bedarf um bis zu 10% zu reduzieren, was zwar einen geringfügigen Leistungsrückgang erzeugt, jedoch die Lebensdauer der Lampe um 60% erhöht. Das komplette Elektroanschlußteil 6 ist wasserdicht gekapselt und mit einem Kühlkörper 12 zur Wärmeabfuhr leitend gekoppelt. Ein weiterer Kühlkörper 10 ist an der dem Patienten 1 abgewandten Seite der Lampenoberfläche, die zusätzlich dort noch einen Reflektor hat, angekoppelt und hält die Lampentemperatur bei 8° C.

Die emittierte Strahlung 2 wird durch einen mit Wasser 7 beaufschlagten Strahlungskühler 11 mit transparenter Abdeckung 4 geleitet, um sodann als Nutzstrahlung 3 für die Hautbehandlung verwendet zu werden.
Die Figuren 1a und b zeigen im Teilschnitt unterschiedliche Ausbildungen der Kühlkörper 12, die ihrerseits am Modul befestigt sein können.

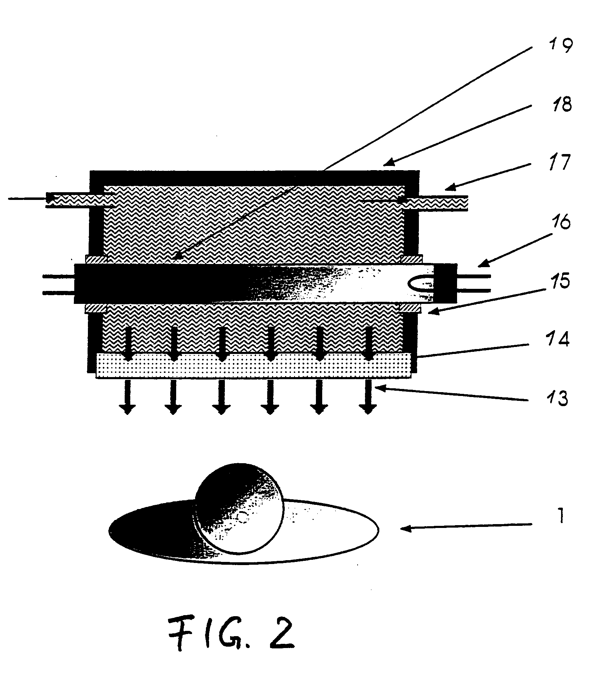

Fig. 2 zeigt in analoger Weise ein Kühlgehäuse 18 für Kühlmittel 17 in den Lampen 19 mit Dichtungen 15 eingelassen sind und umspült werden. Hier ist das komplette Teil als auswechselbares und mit Steckkontakten 16 an eine Stromversorgung anschließbares Modul gestaltet. Die Nutzstrahlung 13 muß eine transparente Abschirmung 14 durchdringen, bevor es den Patienten 1 erreicht.

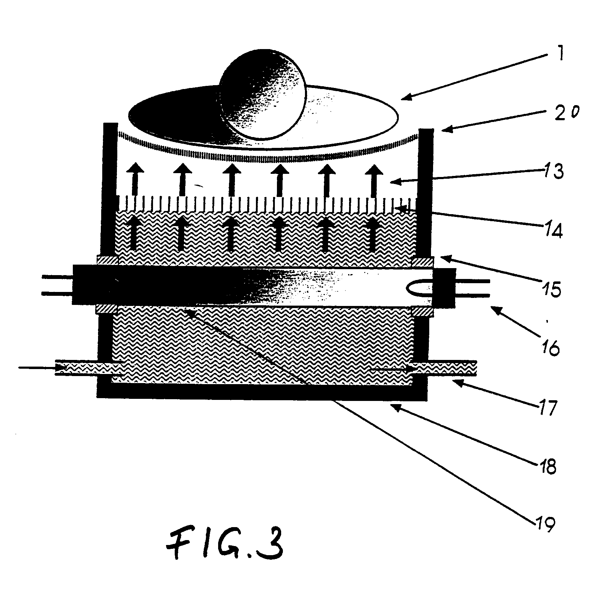

Fig. 3 zeigt ein Standgerät in zu Fig.2 analoger Weise. Der Patient liegt hier auf einer der Hängematte ähnlichen nachgiebigen strahlungsdurchlässigen Matte 20.

## Patentansprüche

1. Behandlungsanordnung für Lichttherapie an Patienten umfassend eine Patientenruheeinrichtung (20), eine Stromversorgung, eine Lichtbestrahlungseinrichtung und eine Kühleinrichtung (10,12,14,15,17,18) für die Lichtbestrahlungseinrichtung, wobei die Lichtbestrahlungseinrichtung im wesentlichen aus parallel angeordneten Leuchtstofflampen (9,19,29) besteht, strahlend in einem nahezu monochromatischen Wellenlängenbereich,
**dadurch gekennzeichnet, daß**
in der Bestrahlungseinrichtung mindestens zwei unterschiedlich strahlende Lampentypen (9,19,29) angeordnet sind, strahlend in unterschiedlichen Wellenlängenbereichen im Spektrum von etwa 290 bis 850 nm, und eine spezifische Strahlungsleistung an einer zu bestrahlenden Projektionsfläche von ≥ 50 mW pro cm² erzeugt wird ,wobei der Hauptanteil der Strahlungsleistung in den ausgewählten Wellenlängenbereichen erbracht wird.

2. Anordnung nach Anspruch 1, dadurch gekennzeichnet, daß die Leuchtstofflampen (9,19,29) der gewählten Wellenlängenbereiche abwechselnd in der Strahlungseinrichtung angeordnet werden.

3. Anordnung nach Anspruch 1 oder 2, dadurch gekennzeichnet, daß die Leuchtstofflampen (9) in auswechselbaren Modulen (21) mit jeweils einer Vielzahl paralleler Lampen (9) angeordnet sind und ein oder mehrere auswechselbare Module (21) eine Bestrahlungseinrichtung bilden.

4. Anordnung nach einem der Ansprüche 1 bis 3, dadurch gekennzeichnet, daß von mehreren Modulen (21) einer Bestrahlungseinrichtung jedes einzelne Modul (21) mit Lampen (9), die in demselben Wellenlängenbereich strahlen, bestückt ist.

5. Anordnung nach einem der Ansprüche 1 bis 4, dadurch gekennzeichnet, daß die Leuchtstofflampen (9,19,21) als induktive Niederdruckgasentladungslampen mit einer Amalgam-Verbindungen enthaltenden Füllung ausgebildet sind, die eine den ausgewählten Wellenlängenbereichen entsprechende, für sich bekannte, Dotierung haben.

6. Anordnung nach einem der Ansprüche 1 bis 5, dadurch gekennzeichnet, daß die emittierende Fläche der Bestrahlungseinrichtung auf mindestens 75 % der Projektionsfläche bemessen wird.

7. Anordnung nach einem der Ansprüche 1 bis 6, dadurch gekennzeichnet, daß die Leuchtstofflampen (9,19,29) an ihrer Oberfläche eine die Strahlung richtende Reflexionseinrichtung aufweisen.

8. Anordnung nach einem der Ansprüche 1 bis 7, dadurch gekennzeichnet, daß zwischen der Projektionsfläche und den Leuchtstofflampen (9 bzw. 19) eine Filtereinrichtung (4,7,11 bzw. 14,17) für Spektralbereiche oberhalb 850 nm und/oder unterhalb 290 nm angeordnet ist.

9. Anordnung nach Anspruch 8, dadurch gekennzeichnet, daß für Infrarotstrahlung Wasser (7,17) als Filter benutzt wird.

10. Anordnung nach einem der Ansprüche 1 bis 9, dadurch gekennzeichnet, daß die Kühleinrichtung (10,12,14,15,17,18) ausgelegt ist auf eine die Oberfläche der Leuchtstofflampen (9,29) auf etwa 5 bis 30° C, vorzugsweise 8 bis 20°C haltende Temperatur.

11. Anordnung nach einem der Ansprüche 1 bis 10, dadurch gekennzeichnet, daß die von der Kühleinrichtung (10,12,14,15,17,18) abgeführte Wärme benutzt wird, mindestens Teile der Patientenruheeinrichtung (20) auf für die Behandlung des Patienten (1) optimale Temperaturen einzustellen.

12. Anordnung nach einem der Ansprüche 1 bis 11, dadurch gekennzeichnet, daß innerhalb eines Modules (21) Leuchtstofflampen (9,19,29) in folgenden Kombinationen eingesetzt werden:
a) 311 nm und 370 nm
b) 380 nm und 620 nm
c) 460 nm und 530 nm
d) 300 nm und 350 nm
e) 300 nm und 350 nm und 380 nm
f) 410 nm und 635 nm
g) 370 nm und 410 nm.

13. Anordnung nach einem der Ansprüche 1 bis 12 dadurch gekennzeichnet, daß die Leuchtstofflampen (9,19,29) mittels wählbarer Stoßionisation bei Frequenzen von 50 Hz bis 200 kHz betreibbar sind.

## Claims

1. Treatment arrangement for light therapy on patients, comprising a means (20), on which a patient may rest, a current supply, a light irradiator and cooling means (10, 12, 14, 15, 17, 18) for the light irradiator, the light irradiator substantially comprising fluorescent lamps (9, 19, 29) disposed in a parallel manner and radiating in a nearly monochromatic wavelength range, characterised in that at least two variably radiating lamp types (9, 19, 29) are disposed in the irradiator, radiating in variable wavelength ranges in the spectrum substantially from 290 to 850 nm, and a specific radiated power of ≥ 50 mW per cm² is produced on a projection area to be irradiated, the main proportion of the radiated power being produced in the selected wavelength ranges.

2. Arrangement according to claim 1, characterised in that the fluorescent lamps (9, 19, 29) of the selected wavelength ranges are disposed alternately in the radiation means.

3. Arrangement according to claim 1 or 2, characterised in that the fluorescent lamps (9) are disposed in interchangeable modules (21), each with a plurality of parallel lamps (9), and one or more interchangeable modules (21) form an irradiator.

4. Arrangement according to one of claims 1 to 3, characterised in that each individual module (21) of the plurality of modules (21) of an irradiator is provided with lamps (9) which radiate in the same wavelength range.

5. Arrangement according to one of claims 1 to 4, characterised in that the fluorescent lamps (9, 19, 29) are configured as inductive low-pressure gas discharge lamps with a filling containing amalgam compounds, said lamps having a doping which is known per se and corresponds to the selected wavelength ranges.

6. Arrangement according to one of claims 1 to 5, characterised in that the emitting area of the irradiator is dimensioned to be at least 75 % of the projection area.

7. Arrangement according to one of claims 1 to 6, characterised in that, on their surface, the fluorescent lamps (9, 19, 29) have a reflection means directing the radiation.

8. Arrangement according to one of claims 1 to 7, characterised in that, for spectral ranges above 850 nm and/or below 290 nm, a filtering means (4, 7, 11 or 14, 17 respectively) is disposed between the projection area and the fluorescent lamps (9 or 19 respectively).

9. Arrangement according to claim 8, characterised in that water (7, 17) is used as the filter for infrared radiation.

10. Arrangement according to one of claims 1 to 9, characterised in that the cooling means (10, 12, 14, 15, 17, 18) is designed for a temperature which keeps the surface of the fluorescent lamps (9, 29) substantially to between 5 and 30° C, preferably between 8 and 20° C.

11. Arrangement according to one of claims 1 to 10, characterised in that the heat discharged from the cooling means (10, 12, 14, 15, 17, 18) is used to set at least parts of the means (20), on which a patient may rest, at temperatures which are optimum for the treatment of the patient (1).

12. Arrangement according to one of claims 1 to 11, characterised in that fluorescent lamps (9, 19, 29) are used in the following combinations inside a module (21):
a) 311 nm and 370 nm
b) 380 nm and 620 nm
c) 460 nm and 530 nm
d) 300 nm and 350 nm
e) 300 nm and 350 nm and 380 nm
f) 410 nm and 635 nm
g) 370 nm and 410 nm.

13. Arrangement according to one of claims 1 to 12, characterised in that the fluorescent lamps (9, 19, 29) are operable by means of selectable ionisation by impact, at frequencies of between 50 Hz and 200 kHz.

## Revendications

1. Système pour photothérapie à pratiquer sur des patients comprenant un agencement pour le repos du patient (20), une alimentation en courant électrique, un agencement d'irradiation de lumière et un agencement de refroidissement (10, 12, 14, 15, 17, 18) pour l'agencement d'irradiation de lumière, l'agencement d'irradiation de lumière se composant essentiellement de lampes fluorescentes (9, 19, 29) agencées en parallèle, irradiant dans un domaine de longueur d'onde presque monochromatique, caractérisé en ce que, dans l'agencement d'irradiation sont agencés au moins deux types de lampes (9, 19, 29) irradiant dans différents domaines de longueur d'onde dans le spectre compris à peu près entre 290 et 850 nm et produisent une puissance d'irradiation spécifique supérieure ou égale à 50 mW par cm2 sur une surface de projection à irradier, la majeure partie de la puissance d'irradiation étant fournie dans les domaines de longueur d'onde choisis.

2. Agencement selon la revendication 1, caractérisé en ce que les lampes fluorescentes (9, 19, 29) des domaines de longueur d'onde choisis sont agencées en alternance dans l'agencement d'irradiation.

3. Agencement selon la revendication 1 ou 2, caractérisé en ce que les lampes fluorescentes (9) sont agencées dans des modules interchangeables (21), chacun avec une pluralité de lampes parallèles (9) et un ou plusieurs modules (21) interchangeables forment un agencement d'irradiation.

4. Agencement selon l'une des revendications 1 à 3, caractérisé en ce que, à partir de plusieurs modules (21) d'un agencement d'irradiation, chaque module individuel (21) est équipé de lampes (9) qui irradient dans le même domaine de longueur d'onde.

5. Agencement selon l'une des revendications 1 à 4, caractérisé en ce que les lampes fluorescentes (9, 19, 29) sont formées comme des lampes à décharge gazeuses à basse pression avec une substance de remplissage contenant des compositions d'amalgame, qui ont un dopage connu en soi correspondant aux domaines de longueur d'onde choisis.

6. Agencement selon l'une des revendications 1 à 5, caractérisé en ce que la surface d'émission de l'agencement d'irradiation est calculée de manière à représenter au moins 75 % de la surface de projection.

7. Agencement selon l'une des revendications 1 à 6, caractérisé en ce que les lampes fluorescentes (9, 19, 29) présentent, sur leur surface, un agencement réfléchissant orientant l'irradiation.

8. Agencement selon l'une des revendications 1 à 7, caractérisé en ce que, entre la surface de projection et les lampes fluorescentes (9 et 19) est agencé un système de filtre (4, 7, 11 et 14, 17) pour des domaines spectraux au-dessus de 850 nm et/ou au-dessous de 290 nm.

9. Agencement selon la revendication 8, caractérisé en ce qu'on utilise comme filtre de l'eau à rayonnement infrarouge (7, 17).

10. Agencement selon l'une des revendications 1 à 9, caractérisé en ce que l'agencement de refroidissement (10, 12, 14, 15, 17, 18) est exposé à une température de surface des lampes fluorescentes (9, 29) maintenant la surface de 5 à 30°C environ, de préférence de 8 à 20°C.

11. Agencement selon l'une des revendications 1 à10, caractérisé en ce qu'on utilise la chaleur évacuée de l'agencement de refroidissement (10, 12, 14, 15, 17, 18), au moins sur des parties de l'agencement pour le repos du patient (20) afin de régler des températures optimales pour le traitement des patients (1).

12. Agencement selon l'une des revendications 1 à 11, caractérisé en ce qu'à l'intérieur d'un module (21), des lampes fluorescentes (9, 19, 29) sont installées selon la combinaison suivante :
a) 311 nm et 370 nm
b) 380 nm et 620 nm
c) 460 nm et 530 nm
d) 300 nm et 350 nm
e) 300 nm et 350 nm et 380 nm
f) 410 nm et 635 nm
g) 370 nm et 410 nm.

13. Agencement selon l'une des revendications 1 à 12, caractérisé en ce que les lampes fluorescentes (9, 19, 29) peuvent être utilisées grâce à une ionisation par choc réglable à des fréquences comprises entre 50 Hz et 200 Hz.
